Europäisches Patentamt

European Patent Office

Office européen des brevets

(19)

(11) **EP 0 901 625 B1**

(12) **EUROPÄISCHE PATENTSCHRIFT**

(45) Veröffentlichungstag und Bekanntmachung des Hinweises auf die Patenterteilung:
**30.07.2003 Patentblatt 2003/31**

(51) Int Cl.$^7$: **G01N 33/18**, G01N 1/14, E02D 1/06, E21B 47/08

(21) Anmeldenummer: **97923885.4**

(22) Anmeldetag: **14.05.1997**

(86) Internationale Anmeldenummer:
**PCT/EP97/02450**

(87) Internationale Veröffentlichungsnummer:
**WO 97/043637 (20.11.1997 Gazette 1997/50)**

(54) **VERFAHREN UND VORRICHTUNGEN ZUR CHARAKTERISIERUNG VON GRUNDWASSERMESSSTELLEN DURCH UNTERSCHEIDUNG VON GRUNDWASSER UND STANDWASSER**

PROCESS AND DEVICES FOR CHARACTERISING GROUND WATER MEASUREMENT POINTS BY DISTINGUISHING GROUND WATER FROM SUBTERRANEAN WATER ACCUMULATION

PROCEDE ET DISPOSITIFS POUR CARACTERISER DES POINTS DE MESURE D'EAUX SOUTERRAINES PAR DIFFERENCIATION DES EAUX SOUTERRAINES ET DES EAUX DORMANTES

(84) Benannte Vertragsstaaten:
**AT BE CH DK ES FI FR GB IE LI NL SE**

(30) Priorität: **14.05.1996 DE 19621158**
**06.03.1997 DE 19711110**

(43) Veröffentlichungstag der Anmeldung:
**17.03.1999 Patentblatt 1999/11**

(73) Patentinhaber:
**UFZ-UMWELTFORSCHUNGSZENTRUM LEIPZIG-HALLE GMBH**
**04318 Leipzig (DE)**

(72) Erfinder:
• **DEHNERT, Joerg**
**D-01819 Berggie hübel (DE)**
• **FREYER, Klaus**
**D-04454 Holzhausen (DE)**
• **NESTLER, Wolfgang**
**D-01277 Dresden (DE)**
• **TREUTLER, Hanns-Christian**
**D-04683 Naunhof (DE)**

(74) Vertreter: **Hengelhaupt, Jürgen, Dipl.-Ing. et al**
**Patentanwälte**
**Gulde Hengelhaupt Ziebig,**
**Büro Berlin-Adlershof**
**Rudower Chaussee 29**
**12489 Berlin (DE)**

(56) Entgegenhaltungen:
DD-A- 282 770          DE-A- 3 911 366
DE-U- 29 704 842

• HOEHN E ET AL: "Radon in groundwater: a tool to assess infiltration from surface waters to aquifers" WATER RESOURCES RESEARCH, AUG. 1989, USA, Bd. 25, Nr. 8, ISSN 0043-1397, Seiten 1795-1803, XP002040816
• WILLME U ET AL: "BESTIMMUNG VON GRUNDWASSERVERWEILZEITEN MIT GEOGENEM 222RADON BEI KUNSTLICHER GRUNDWASSERANREICHERUNG UND UFERFILTRATION IN EINER TRINKWASSERGEWINNUNGSANLAGE" GWF WASSER ABWASSER, Bd. 136, Nr. 5, 1.Mai 1995, Seiten 234-241, XP000506703
• LABED V ET AL: "Study of /sup 222/Rn permeation through polymer membranes: application to continuous measurement of /sup 222/Rn in water" HEALTH PHYSICS, AUG. 1992, USA, Bd. 63, Nr. 2, ISSN 0017-9078, Seiten 172-178, XP002040817
• PATENT ABSTRACTS OF JAPAN vol. 095, no. 002, 31.März 1995 & JP 06 324200 A (NITSUSAKU:KK), 25.November 1994,

EP 0 901 625 B1

**Beschreibung**

Die Erfindung betrifft ein Verfahren zur Bestimmung der optimalen Abpumpzeiten von Grundwassermeßstellen durch Meßwerterfassung von Grundwassermeßstellenparametern und dient sowohl der Vor-Ort-Bestimmung des optimalen Probenahmezeitpunktes, der Bestimmung der für repräsentative Beschaffenheitsuntersuchungen optimalen Abpumpzeiten sowie der Überwachung von Grundwassermeßstellen auf Defekte.
Die Erfindung ist anwendbar insbesondere im Rahmen von Beschaffenheitsuntersuchungen des Grundwassers.
Sowohl der Schutz des Grundwassers zur Trinkwassergewinnung als auch die Überprüfung von Altlastenverdachtsflächen, die Altlastenuntersuchung und die Sanierung von Schadensfällen machen die Gewinnung einer ständig steigenden Anzahl von Grundwasserproben notwendig. Um das Grundwasser zu beproben, wurden und werden Grundwasserbeobachtungsrohre bzw. Grundwassermeßstellen errichtet. Gleichzeitig wurde die Analytik zur Bestimmung der Wasserinhaltsstoffe verfeinert und das Spektrum der nachweisbaren Einzelstoffe ausgeweitet. Beides führte zu einer neuen Qualitätsanforderung an die Grundwasserprobe. Die hohen Anforderungen der Analytik und der finanzielle Aufwand zur Gewinnung einer Grundwasserprobe verlangen gleichermaßen eine sorgfältige, teufenorientierte und repräsentative Probennahme. Voraussetzung hierfür ist die richtige Auswahl von Meßstellentyp, Beprobungstechnik und Probenahmetechnologie. Darüberhinaus ist für die Repräsentanz einer Grundwasserprobe die Kenntnis der optimalen Abpumpzeit maßgeblich. Wenn eine Probe wegen einer zu kurz gewählten Abpumpzeit größere Anteile Standwasser aus dem Grundwasserbeobachtungsrohr enthält, kann die Bewertung des Analysenergebnisses zu falschen Schlußfolgerungen führen. Eine zu lange Abpumpzeit hingegen kann Wasserkörper anderer Horizonte heranziehen, was ebenfalls unerwünscht ist. Die richtige Bestimmung der Abpumpzeiten für Grundwasserbeobachtungsrohre ist ein bisher nicht vollständig gelöstes Problem.
Vor jeder Probennahme muß das zu beprobende Grundwasserbeobachtungsrohr abgepumpt werden, bis das geförderte Wasser dem des umgebenden Grundwassers entspricht und nicht mehr durch die Meßstelle beeinflußt wird. Hierzu ist es bekannt, bis zur Konstanz von elektrischer Leitfähigkeit, Temperatur und pH-Wert abzupumpen. Gleichzeitig ist bekannt, daß die elektrische Leitfähigkeit nur eine Orientierungsgröße ist. Weitere bekannte Methoden sind Faustformeln wie der mehrfache Austausch des Rohrinhaltes. Zusammenfassend kann eingeschätzt werden, daß die Konstanz der elektrischen Leitfähigkeit heute das allgemein übliche Kriterium für die Bestimmung des Zeitpunktes einer repräsentativen Probennahme ist, obwohl über die Prozesse, die das Abfallen der elektrischen Leitfähigkeit in einem Grundwasserbeobachtungsrohr verursachen, nur wenig bekannt ist.
Nachteilig an dieser Methode ist, daß diese Konstanz zwar als notwendige Bedingung für eine nachfolgende Probennahme betrachtet werden muß, sie ist aber nicht hinreichend, da die elektrische Leitfähigkeit auch vor dem Zeitpunkt für die repräsentative Probennahme einen Plateauwert erreichen kann. Das tritt beispielsweise auf, wenn das Grundwasserbeobachtungsrohr Tage oder Wochen vor der Probennahme schon einmal beprobt worden ist. Die Radonaktivitätskonzentration hingegen kann als zuverlässiges Kriterium auch dann noch eingesetzt werden, wenn zwischen zwei Beprobungen nur wenige Tage liegen.
Außerdem ist die relative Änderung zwischen dem Ausgangswert (Standwasser) und dem Endwert (Grundwasser) bei der Leitfähigkeit in der Regel um ein Vielfaches kleiner als bei der Radonaktivitätskonzentration, was das Festlegen des optimalen Zeitpunktes für die repräsentative Probennahme bei Verwendung der Radonaktivitätskonzentration erleichtert.
Ein derartiges Verfahren zur Bestimmung der optimalen Abpumpzeiten von Grundwasserbeobachtungsrohren zur Festlegung eines optimalen Probenahmezeitpunkts ist aus der DE 3911366 C2 bekannt, bei welchem die Leitfähigkeit des Wassers überwacht und bei konstanter Leitfähigkeit die repräsentative Probenahme eingeleitet wird.
Weiterhin wurde mit der DE 42 17 263 A1 eine Lösung bekannt, mit welcher bei der Probenahme auch gasförmige Bestandteile überwacht werden.
Allerdings handelt es sich bei dieser Lösung nicht darum, Gas zur Festlegung des Probenahmezeitpunktes zu nutzen, sondern um den zur GW-Probenahme analogen Vorgang der Gasprobenahme.
In HOEHN E ET AL: "Radon in groundwater: a tool to assess infiltration from surface waters to aquifers" WATER RESOURCES RESARCH, AUG. 1989, USA, Bd. 25, Nr. 8, ISSN 0043-1397, Seiten 1795-1803, XP002040816 wird Radon als natürlicher Tracer im Grundwasser zur Bestimmung von Aufenthaltszeiten von Uferfiltrat beschrieben, d.h. es wird die Laufzeit bestimmt, die das in einem Fluß infiltrierte Wasser bis zu einer Grundwassermeßstelle benötigt. Grundidee ist dabei, daß radonarmes Flußwasser bei seinem Weg durch den Grundwasserleiter Radon aufnimmt, bis sich eine Sättigungskonzentration einstellt. Zur Bestimmung der Abpumpzeit in Grundwassermeßstellen wurde Radon nicht verwendet.
Aus den Literaturstellen Health Physics, Vol. 53 (1987) S. 181-186 und Radioisotopes, Vol. 30 (1981) S. 649-654 schließlich sind Verfahren zur Messung von Radon in Luft (ungesättigte Zone, Bodenluft) mittels LSC-Verfahren bekannt. Hier wird radonhaltige Luft mit einem Einleitungsrohr in stehendes Wasser eingeblasen, damit es sich im Wasser löst und dann aus dem Wasser mit einem LSC-Cocktail wieder extrahiert und gemessen werden kann.
Der Erfindung liegt die Aufgabe zugrunde, ein Verfahren zur Bestimmung der für repräsentative Beschaffenheitsuntersuchungen optimalen Abpumpzeiten von Grundwasserbeobachtungsrohren zu schaffen, welches es gestattet, mit vertretbarem Aufwand und hoher Genauigkeit zuverlässig und reproduzierbar den optimalen Abpumpzeitpunkt zu bestimmen und eine sichere Bestimmung des Verhältnisses von Grundwasser zu Standwasser in einer Grundwasserprobe sowie die Lokalisierung von Defekten zu gewährleisten.

Diese Aufgabe wird erfindungsgemäß gelöst durch die Merkmale im kennzeichnenden Teil der Ansprüche 1, 13 und 16.

Zweckmäßige Ausgestaltungen der Erfindung sind in den Unteransprüchen enthalten.

Ein besonderer Vorteil der Erfindung besteht darin, daß die Bestimmung der optimalen Abpumpzeiten beziehungsweise des optimalen Zeitpunktes für die Probenentnahme mit sehr hoher Genauigkeit und Reproduzierbarkeit erfolgt, indem während des Abpumpvorganges in definierter zeitlicher Aufeinanderfolge Wasserproben entnommen werden, von den entnommenen Proben die Radonaktivitätskonzentration gemessen wird und das Erreichen einer im wesentlichen konstant bleibenden Radonaktivitätskonzentration den optimalen Zeitpunkt für repräsentative Probennahmen signalisiert.

Ein weiterer Vorteil der Erfindung besteht darin, daß die Erfassung der Grundwassermeßstellenparameter ohne Zeitverzug erfolgen kann, indem direkt aus dem zu untersuchenden Grundwasser vor Ort die Radonaktivitätskonzentration und/oder die Gesamtaktivitätskonzentration spektrometrisch und/oder durch Diffusion in ein Luftvolumen oder Ausgasen von nachfolgender Messung erfaßt und ausgewertet wird.

Die Erfindung soll nachstehend anhand von teilweise in den Figuren dargestellten Ausführungsbeispielen näher erläutert werden. Es zeigen:

Fig. 1    die Verteilung der Radonaktivitätskonzentration an einem Grundwasserbeobachtungsrohr

Fig. 2    eine schematische Darstellung des Probenahmegefäßes mit Einleitungsrohr

Fig. 3    ein Diagramm der gemessenen Radonaktivitätskonzentration und elektrischen Leitfähigkeit während eines Abpumpversuches mit Pumpe direkt unter der Grundwasseroberfläche

Fig. 4    ein Diagramm der gemessenen Radonaktivitätskonzentration und elektrischen Leitfähigkeit während eines Abpumpversuches mit Pumpe im Filterbereich

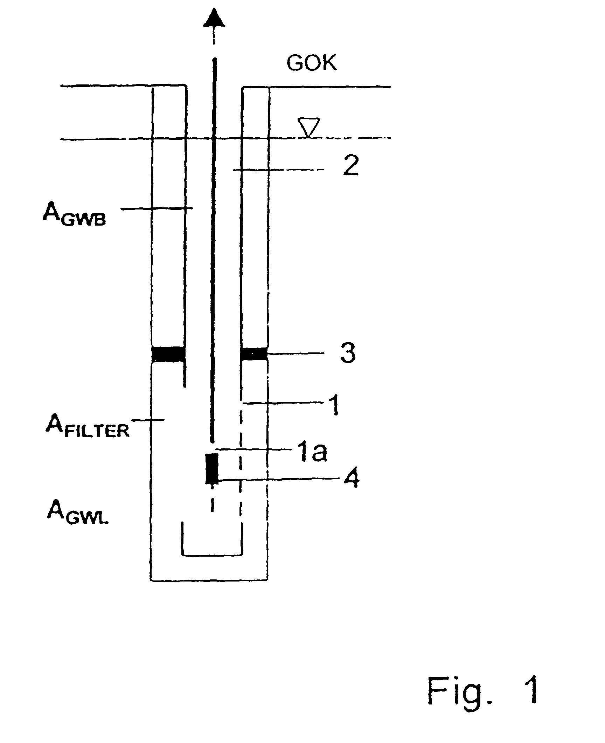

Wie aus Figur 1 zu ersehen ist, treten im Bereich eines Grundwasserbeobachtungsrohres im Grundwasser drei unterschiedliche Radonaktivitätskonzentrationen auf. Das Korngerüst des Grundwasserleiters produziert eine Basisaktivität $A_{GWL}$. Der eingebaute Filterkies 1 mit fremder Herkunft und größerem Korndurchmesser produziert seine eigene Radonaktivitätskonzentration $A_{Filter}$. Die Aktivität im nicht verfilterten Standrohr 2 des Grundwasserbeobachtungsrohres $A_{GWBR}$ hingegen ist null.

Der Porenraum des Filterkieses 1 unterhalb der Tonsperre 3 und das Filterrohr 1a des Grundwasserbeobachtungsrohres werden vom Grundwasser mit der Aktivität $A_{GWL}$ durchströmt. In Abhängigkeit von der Strömungsgeschwindigkeit des Grundwassers und der eingebauten Menge des Filterkieses 1 dominiert die Aktivität des Grundwassers auch im Filterkies 1 und im Innenraum des Filterrohres 1a. Im unverfilterten Standrohr (Aufsatzrohr) 2 des Grundwasserbeobachtungsrohres hingegen wird kein Radon gebildet. Die Aktivität des Wassers im nicht durchströmten Standrohr 2 sinkt deshalb entsprechend der Gleichung für den radioaktiven Zerfall auf den Wert null ab:

$$A_t = A_e \, e^{-\lambda t}$$

mit

$$A_t = \text{Radonaktivität zum Zeitpunkt } t \tag{1}$$

$$A_e = \text{Radonaktivität im Gleichgewicht}$$

$$\lambda = \text{radioaktive Zerfallskonstante, } I_{Rn} = 0{,}18 \, d^{-1}$$

Dies bedeutet: Wenn durch eine Probennahme in das Standrohr 2 Grundwasser mit der Aktivität $A_{GWL}$ gepumpt wurde, kann wegen der kurzen Halbwertszeit des Radons nach einer Standzeit von 26 Tagen nur noch eine Aktivität von 1% des Ausgangswertes gemessen werden.

Damit ist Radon ein idealer Parameter zur Bestimmung des Standwasseranteils in einer Grundwasserprobe. Mißt man die Radonaktivitätskonzentration des abgepumpten Wassers, so beginnt diese bei null und nähert sich entsprechend dem Mischungsverhältnis von Grund- und Standwasser einem Plateauwert. Anhand einer solchen Kurve kann der Zeitpunkt für eine repräsentative Probenahme als Funktion der Austauschvolumina des Grundwasserbeobachtungsrohres exakt bestimmt werden.

Bei allen Untersuchungen zum Abpumpverhalten eines Grundwasserbeobachtungsrohres müssen die beiden Möglichkeiten des unterschiedlichen Pumpeneinbaus im Filterbereich oder direkt unterhalb der Grundwasseroberfläche getrennt betrachtet werden. Möglich ist der Einbau der Pumpe 4 ein Meter unter der Filteroberkante beziehungsweise in Filtermitte oder bei tiefen Grundwasserbeobachtungsrohren und dem Fehlen der dafür notwendigen Pumpentechnik der Einbau der Pumpe 4 einen Meter unterhalb der Grundwasseroberfläche. Durchgehend verfilterte Grundwasserbeobachtungsrohre sind von den folgenden Betrachtungen ausgenommen.

Radon ist ein radioaktives Edelgas und hat drei Isotope mit den Massenzahlen 219, 220 und 222. Sie sind Glieder der natürlichen Zerfallsreihen von $^{238}$U, $^{232}$Th und $^{235}$U. Entscheidend für das Vorkommen und die Verbreitung der drei Radonisotope im Wasser sind ihre Halbwertszeiten. Wenn sie zu kurz sind, zerfallen die Isotope am Ort ihrer Entstehung. So gelangen Thoron ($^{220}$Rn) mit 56 s Halbwertszeit und Actinon ($^{219}$Rn) mit 4 s Halbwertszeit kaum oder gar nicht in die bewegte flüssige Phase. Radon-222 mit 3,8 d Halbwertszeit und seine Folgeprodukte sind deshalb die Hauptquellen der natürlichen Strahlenbelastung des Grundwassers.

Radon-222 (im folgenden als Radon bezeichnet) entsteht aus Radium-226, einem Zerfallsprodukt des $^{238}$U. Die Folgeprodukte des Radons sind Isotope der Elemente Polonium, Wismut und Blei. Der Zerfall des Radons bis zum $^{214}$Po erfolgt durch drei Alpha- und zwei Beta-Zerfälle. Das Gleichgewicht zwischen Radon und seinen Folgeprodukten ist nach ca. drei Stunden erreicht.

Radon tritt insbesondere durch Rückstoßeffekte beim Alpha-Zerfall aus Fest- und Lockergesteinen aus oder es gelangt durch Diffusion an der Kornoberfläche in die flüssige Phase. Es schließt sich ein wegen der kurzen Halbwertszeit begrenzter Transportprozeß durch Diffusion und die Grundwasserströmung (Migration) an.

Das Korngerüst des Grundwasserleiters produziert permanent Radon und gibt dieses an die flüssige Phase ab.

Die Emanationsrate von Lockergesteinen hängt neben der Konzentration des Vorgängerisotops Radium-226 auch von der Korngröße und der Form der Kornoberfläche ab. Die Radonaktivitätskonzentrationen des Grundwassers korrelieren dabei mit der Stratigraphie des Grundwasserleiters.

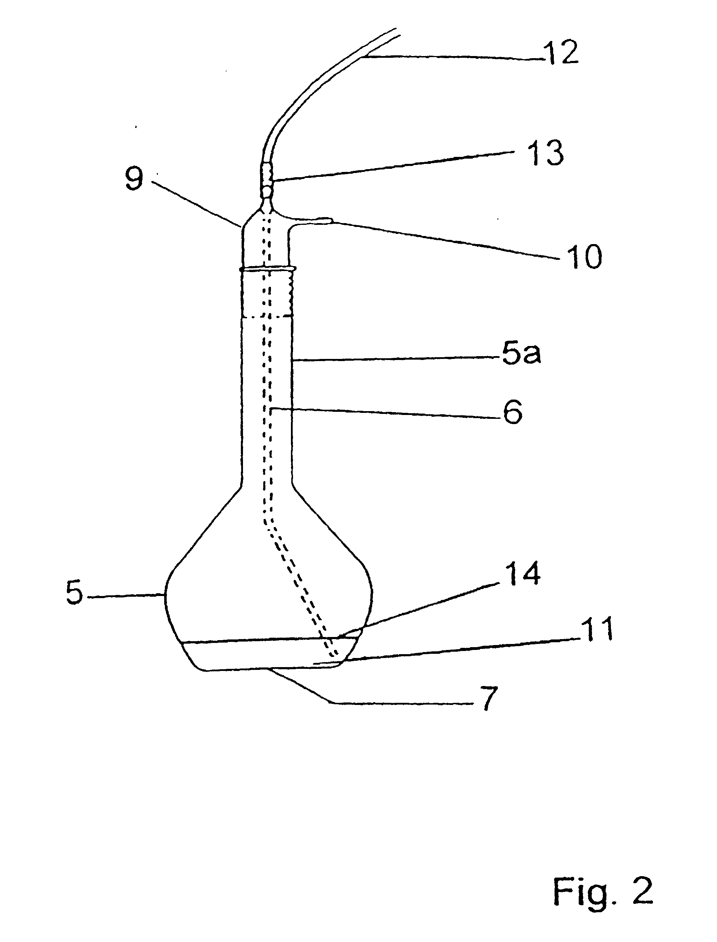

Nachfolgend soll das in Figur 2 dargestellte Probenahmegefäß 5 sowie die Meßtechnologie näher beschrieben werden.

Das Probenahmegefäß 5 ist so geschaffen, daß beim Befüllen und beim anschließenden Transport das sehr mobile Radon nicht entweichen kann. Vor allem darf die Wasserprobe nicht mit Luft in Kontakt kommen. Realisiert wird daher eine turbulenzarme Unterschichtung ohne Luftkontakt des auf der Grundwasserprobe 11 schwimmenden Cocktails 14 beim Befüllen des Probenahmegefäßes 5. Um dies zu gewährleisten, ist ein Einleitungsrohr 6 mit Schliffkern in einem Aufsatz 9 angeordnet, welcher auch ein Entlüftungsrohr 10 aufweist, wobei sich das Einleitungsrohr 6 in das Probenahmegefäß 5 hinein bis kurz oberhalb des Probenahmegefäßbodens 7 erstreckt und das Ende des Einleitungsrohres 6 sich unter der Oberfläche eines Toluen-Szintillators befindet. Zu Beginn der Probennahme wird das Probenahmegefäß 5 in der Weise schräg gehalten, daß sich der Cocktail 14 am Ende des Einleitungsrohres 6 sammelt und eine ausreichende Überschichtung der einströmenden Grundwasserprobe auch während der Anfangsturbulenzen gesichert ist. Das Probenvolumen beträgt im vorliegenden Ausführungsbeispiel ein Liter. Nach der Probennahme wird das Einleitungsrohr 6 aus dem Probenahmegefäß 5 entfernt und das Probenahmegefäß 5 mit einem Stopfen aus Polyethylen fest verschlossen. Zusätzlich werden zwischen dem Stopfen und dem Hals 5a Dichtungen aus Teflon angeordnet.

Wegen der bezüglich Wasser um das Vielfache höheren Löslichkeit von Radon in dem Toluen-Szintillator-Cocktail, bildet der im engen Hals 5a des Probenahmegefäßes 5 schwimmende Cocktail 14 einen sicheren Schutz gegen Radonverluste beim Transport der Probe.

Um Radonverluste schon bei der Probennahme zu vermeiden und gleichzeitig die Probenahmebedingungen objektiver zu gestalten, wird der Aufsatz 9 mit Entlüftungsrohr 10 sowie das Einleitungsrohr 6 mit Schliffkern aus Glas gefertigt. Der Cocktail 14 wird bereits vor der Probennahme in das Probenahmegefäß 5 gegeben und mit Hilfe des Einleitungsrohres 6 von der Grundwasserprobe 11 unterschichtet. Beim Einsatz der Unterwasserpumpe 4 wird über einen Bypass und einen Polyethylenschlauch 12 und bei den Membranpumpen über eine direkte Ankopplung des Polyethylenschlauches 12 über eine Schlauchkupplung 13 die Grundwasserprobe 11 ohne Kontakt mit der Luft direkt über das Einleitungsrohr 6 unter den Cocktail 14 geleitet. Zum Schutz des gläsernen Einleitungsrohres 6 vor Beschädigungen kann eine zweite Schlauchkupplung derart eingesetzt werden, daß das Einleitungsrohr 6 mit einem Stück Schlauch über die Schlauchkupplung 13 fest verbunden bleibt und die Ankopplung und Trennung des Polyethylenschlauches 12 über eine zweite Schlauchkupplung erfolgt. Die Probennahme erfolgt so turbulenzarm und blasenfrei.

Die mit der Unterwassermotorpumpe 4 gewonnene Wasserprobe wird über einen Bypass und ein Einleitungsrohr blasenfrei und ohne Luftkontakt in einen Maßkolben 5 mit einem Volumen von 1 Liter gefördert. In diesem Maßkolben 5 befinden sich 20 ml eines Toluen-Szintillators (Cocktail, Toluene Scintillator der Firma Packard, 5 g PPO und 0,1 g POPOP pro Liter Toluen), der von der Grundwasserprobe unterschichtet wird. Im Labor wird das Radon durch Schütteln des Maßkolbens 5 extrahiert und der abpipettierte Cocktail in Vials gegeben. Die Messung der Vials erfolgte mit einem Flüssigszintillationsspektrometer TRI-CARB 2550 TR/AB der Fa. Packard. Zur Beurteilung der Richtigkeit der Meßergebnisse kann die Alpha/Beta-Diskriminierung herangezogen werden. Die Radonkonzentration zum Zeitpunkt der Probennahme wird durch Regression aus mehrfachen Messungen eines Vials berechnet. Die im Flüssigszintillationsspektrometer ermittelten Radonkonzentrationen werden in cpm (counts per minute) angegeben.

Zur Messung der elektrischen Leitfähigkeit und gegebenenfalls des pH-Wertes und der Temperatur werden Meßsonden und eine Durchflußmeßzelle eingesetzt. Der geförderte Grundwasserstrom wird über einen Bypass in zwei Teilströme zur Gewinnung der Grundwasserproben und zum Durchströmen der Durchflußmeßzelle aufgeteilt. Es erfolgte keine Drosselung des Förderstromes während der Probennahme, da die Radonaktivitätskonzentration vom Förderstrom unabhängig ist.

Nachfolgend sollen zwei Abpumpversuche näher erläutert werden. Die zwei beprobten Grundwasserbeobachtungsrohre waren baugleich. Sie hatten einen Rohrdurchmesser von 4,5" bei einem Bohrdurchmesser von 13" und waren mit einem 1 m-langen Edelstahlwickelfilter ausgebaut. Die Korngröße des Filterkieses betrug 2-8 mm, die Porosität wurde mit 0,25 angenommen. Unter Austauschvolumen wird im folgenden das Volumen des Grundwasserbeobachtungsrohres einschließlich des Porenraumes der Kiesfilterschüttung verstanden.

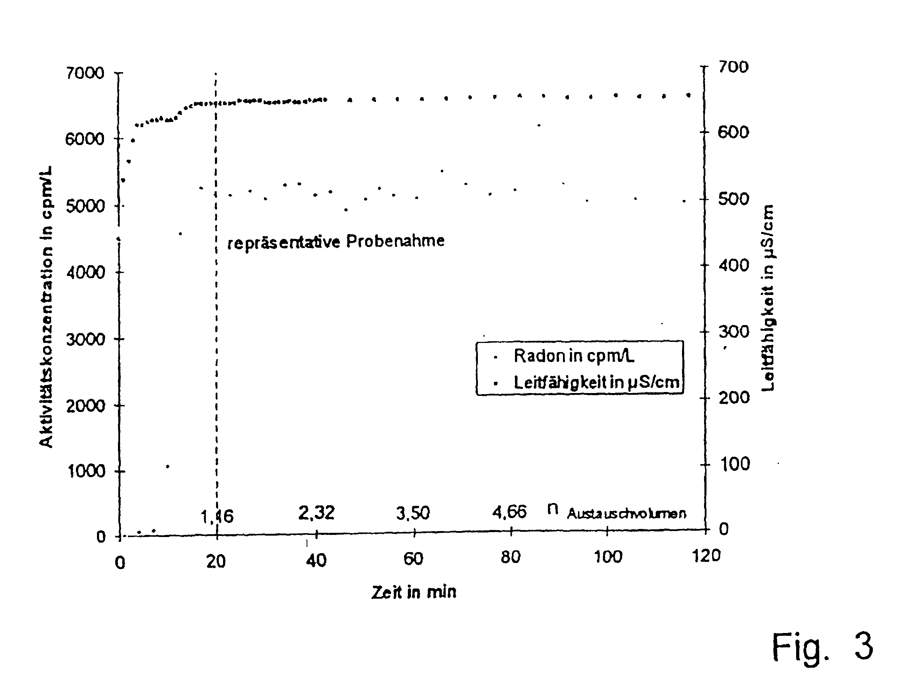

Im Versuch A wurde die Pumpe 4 einen Meter unter der Grundwasseroberfläche (Einbautiefe 6 m) in ein Grundwasserbeobachtungsrohr mit einer stehenden Wassersäule bis zur Filterunterkante von 39 m eingebaut. Die Filterkiesschüttung war 5,5 m mächtig. Bei konstanter Förderleistung der Pumpe von 1,75 m$^3$/h wurde im Abstand von drei Minuten jeweils eine Probe zur Bestimmung der Radonaktivitätskonzentration genommen. Gleichzeitig wurde die elektrische Leitfähigkeit minütlich aufgezeichnet. Der Versuch dauerte 120 min (Fig 3).

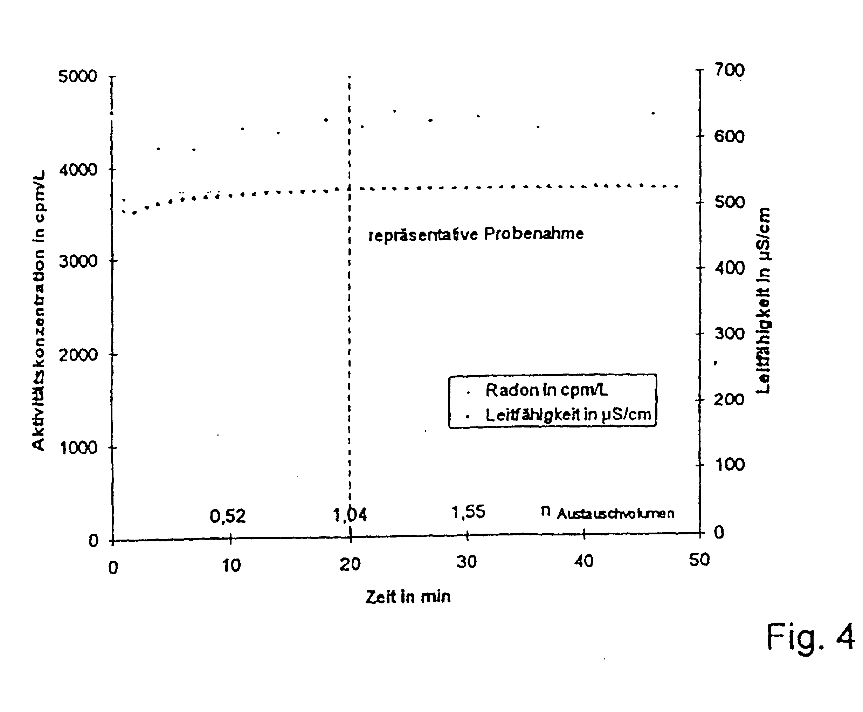

Im Versuch B wurde die Pumpe im Filterbereich des zweiten Grundwasserbeobachtungsrohres installiert (Einbautiefe 48 m, stehende Wassersäule bis zur Filterunterkante 43 m). Die Kiesschüttung war 6,5 m mächtig, die Pumpenleistung betrug 1,73 m$^3$/h. Die Probennahme erfolgte wie beim Versuch A bei einer Versuchsdauer von 50 min (Fig. 4).

Die Auswertung ergab, daß bei Versuch A das gesamte Standwasser im Grundwasserbeobachtungsrohr und im Filterkies vor der repräsentativen Probennahme ausgetauscht werden mußte. Die Radonaktivitätskonzentration begann erwartungsgemäß bei Null und stieg bis zu einem Plateauwert von 5180 cpm/l an (Fig. 3). Diese Kurve ist ein sicheres Maß für das Verhältnis von Standwasser zu Grundwasser in der Probe. Eine repräsentative Grundwasserprobe konnte bereits nach 1,2 Austauschvolumina entnommen werden. Der Anstieg der elektrischen Leitfähigkeit erfolgte deutlich vor dem Anstieg der Radonaktivitätskonzentration.

Bei Versuch B kam es unmittelbar nach dem Einschalten der Pumpe zur Förderung von Grundwasser. Aus der Radonaktivitätskonzentration konnte wieder direkt das Verhältnis von Grundwasser zu Standwasser in der Probe bestimmt werden. Danach enthielt bereits die erste Probe 82% Grundwasser. Die Zumischung des Standwassers bei dieser Anordnung erstreckte sich trotzdem über einen längeren Zeitraum, der normiert auf das Austauschvolumen (hier eine fiktive Rechengröße) bei 1,0 eine repräsentative Probennahme gestattete (Plateau der Radonaktivitätskonzentration bei 4470 cpm/l). Die Leitfähigkeit zeigte bei dieser Anordnung nur eine geringe Zunahme von 30 μS/cm bis zum Plateauwert von 525 μS/cm.

Mit Hilfe der Radonaktivitätskonzentration konnte der Zeitpunkt für die repräsentative Probennahme für ein Grundwasserbeobachtungsrohr mit eingebauter Pumpe 4 direkt unter der Grundwasseroberfläche und ein zweites Grundwasserbeobachtungsrohr mit eingebauter Pumpe 4 im Filterbereich sicher bestimmt werden. Dabei war die relative Amplitudenänderung der Radonaktivitätskonzentration in beiden Versuchen dreimal größer als die der elektrischen Leitfähigkeit (100% zu 32% im Versuch A und 18% zu 6% im Versuch B).

Die Messung der Radonaktivitätskonzentration während des Abpumpvorganges gestattet die sichere Bestimmung des Verhältnisses von Grundwasser zu Standwasser in einer Grundwasserprobe. Ursache ist der schnelle Zerfall des Radons im Standwasser eines Grundwasserbeobachtungsrohres aufgrund seiner kurzen Halbwertszeit in Verbindung mit der ständigen Radonemanation im Korngerüst des Grundwasserleiters. Damit besteht die Möglichkeit, das Abpumpverhalten von Grundwasserbeobachtungsrohren allgemein zu untersuchen und zwar für beliebige Fälle des möglichen Pumpeneinbaues.

Darüberhinaus kann die Radonaktivitätskonzentration genutzt werden, um objektspezifisch Abpumpzeiten für Grundwasserbeobachtungsrohre zu bestimmen. Das erscheint für die Fälle sinnvoll, in denen keine Klarheit über die genaue Abpumpzeit herrscht und dem Ergebnis einer Wasseranalyse besondere Bedeutung zukommt, beispielsweise als Nachweis für den Erfolg einer kostenintensiven Sanierungsmaßnahme. Denkbar wäre auch der Einsatz der Radonaktivitätskonzentration für Grundwasserbeobachtungsrohre der Ländermeßnetze, wo ein hohes Maß an Repräsentanz erforderlich ist und Aufwand und Nutzen in einem ausgezeichneten Verhältnis zueinander stehen. Auch bei der Bewertung der Funktionstüchtigkeit von Alt-Grundwasserbeobachtungsrohren kann die Verwendung der Radonaktivitätskonzentration sinnvoll sein. Die Filter älterer Grundwasserbeobachtungsrohre verokkern, was die hydraulische Funktionstüchtigkeit beeinträchtigt. Die Filter werden nicht mehr oder nur noch wenig vom Grundwasser durchströmt. Zur Bewertung der hydraulischen Funktionstüchtigkeit kann eine Schöpfprobe aus dem Filterbereich entnommen werden. Ist die Radonaktivitätskonzentration dieser Probe Null, wird der Filter nicht mehr vom Grundwasser durchströmt. Wird nach dem Entnehmen der Schöpfprobe ein Abpumpversuch durchgeführt und die Radonaktivitätskonzentration während des Abpumpversuches gemessen, kann aus dem Verhältnis der Radonaktivitätskonzentrationen der Schöpfprobe zum Plateauwert des Versuches auf die Durchströmung des Filters geschlossen werden.

In einer Variation der Erfindung ist es möglich, daß statt der Radonaktivitätskonzentration die Gesamtaktivitätskonzentration gemessen wird, wobei die Radonaktivitätskonzentration einen großen Beitrag zur Gesamtaktivitätskonzentration liefert und andere Nuklide mit größeren Halbwertszeiten das Verfahren nicht stören. Ebenso ist es möglich, daß die Messung der Radonaktivitätskonzentration on-Line erfolgt.

Für die Praxis ist die Vor-Ort-Bestimmung des Probennahmezeitpunktes durch Online-Messung von entscheidender Bedeutung. Bei entsprechenden Überlegungen zur praktischen Anwendbarkeit des Verfahrens muß berücksichtigt werden, daß es eigentlich nicht auf die quantitative Bestimmung der Radonaktivitätskonzentration ankommt, sondern daß die Bestimmung der Gesamtaktivitätskonzentration des Grundwassers für das vorgeschlagene Verfahren ausreicht. Das ist darin begründet, daß Radon den Hauptteil dieser Aktivität verursacht und eine gleichbleibende Hintergrundaktivität von Nukliden mit längeren Halbwertszeiten das Verfahren nicht stört. Demzufolge sind zwei Möglichkeiten für eine Online-Messung zur Bestimmung des Abpumpvolumens von Grundwassermeßstellen möglich:

a) Direkte Messung von Radon und seinen kurzlebigen Folgeprodukten

Die kurzlebigen Polgeprodukte des Radon-222 sind die Radionuklide Po-218, Pb-214, Bi-214 und Po-214, wie aus Tabelle 1 zu ersehen ist.

Tab. 1:

| Zusammenfassung wesentlicher strahlungsphysikalischer Eigenschaften von Rn-222- und Rn-220-Zerfallsprodukten | | | | |
|---|---|---|---|---|
| Isotop | $T_{1/2}$ | Strahlung | $\alpha$-Energie [MeV] | $\gamma$-Energie [MeV] |
| Ra-226 | 1602 a | $\alpha, \gamma$ | 4,78 (94,3 %)<br>4,69 (5,7 %) | 0,186 (3,3 %) |
| **Rn-222** | **3,82 d** | $\alpha$ | **5,49(100 %)** | |
| Po-218 | 3,05 min | $\alpha$ | 6,00(100 %) | |
| Pb-214 | 26,8 min | $\beta,\gamma$ | - | 0,295 (19 %)<br>0,352 (36 %) |
| Bi-214 | 19,7 min | $\beta,\gamma$ | - | 0,609 (47 %)<br>1,120 (15 %) |
| Po-214 | 164 ms | $\alpha$ | 7,68 (100 %) | |
| Pb-210 | 22,3 a | $\beta$ | - | 0,0465 (4,06 %) |
| Ra-224 | 3,66 d | $\alpha$ | 5,45 (6 %)<br>5,68 (94 %) | 0,241 (3,9 %) |
| **Rn-220** | **55 s** | $\alpha$ | **6,29 (100 %)** | |
| Po-216 | 0,15 s | $\alpha$ | 6,78 (100%) | |
| Pb-212 | 10,6 h | $\beta,\gamma$ | - | 0,239 (47 %)<br>0,300 (3,2 %) |
| Bi-212 | 60,6 min | $\alpha, \beta, \gamma$ | 6,05 (25 %)<br>6,09 (10 %) | 0,727 (11,8 %)<br>1,620 (2,8 %) |
| Po-212 | 304 ns | $\alpha$ | 8,78 (100 %) | |
| Tl-208 | 3,1 min | $\beta,\gamma$ | - | 0,511 (23 %)<br>0,583 (86 %)<br>0,860 (12 %)<br>2,614 (100 %) |

Nachdem das Radon aus dem Wasser extrahiert und damit von diesen Folgeprodukten getrennt worden ist, bestimmt im wesentlichen das Folgenuklid mit der längsten Halbwertszeit (Pb-214 mit $T_{1/2}$ = 26,8 min) die Zeit bis zum erneuten Erreichen des radioaktiven Gleichgewichtes nach vier Halbwertszeiten, d.h. nach etwa zwei Stunden. Eine schnelle Messung von Radon ist über eine alphaspektrometrische Bestimmung von Radon und Po-218 ($T_{1/2}$ = 3,1 min) möglich, da sich das Gleichgewicht zwischen Rn-222 und Po-218 schon nach etwa 12 min eingestellt hat. Eine andere Möglichkeit besteht darin, einen aus einer diffussionsdurchlässigen Membran bestehenden, luftgefüllten Schlauch in Form einer Spule in den Förderstrom einer Pumpe einzubauen. Das Radon diffundiert aus dem Wasser durch den Schlauch in die Luft und kann in einer Meßzelle (z.B. Lucas-Zelle) online gemessen werden (SURBECK, 1996). Hier findet sich auch ein Beispiel für eine Online-Messung von Radon im Leitungswasser mit einer zeitlichen Auflösung von 30 min. Eine weitere Reduzierung der Meßzeit auf 1 min, hängt davon ab, ob die dann noch erreichbare Genauigkeit für das vorgeschlagene Verfahren zur Bestimmung von Abpumpzeiten ausreichend ist.

b) Messung der Gesamtaktivitätskonzentration Hier besteht die Möglichkeit, verschiedene bereits existierende Meßgeräte und Meßprinzipien zu nutzen.

Zur Online-Erfassung der Leitkennwerte wird eine neue Durchflußmeßzelle vorgeschlagen, die neben der Messung der natürlichen Radonaktivitätskonzentration auch den Durchfluß und die traditionellen Leitkennwerte elektrische Leitfähigkeit, Temperatur und pH-Wert erfaßt. Alle Leitkennwerte werden als Funktion des Abpumpvolumens durch ein zugehöriges Software-Programm online dargestellt und dokumentiert. Die Auswertung der aufgezeichneten Kurven und insbesondere die Bestimmung der Plateauwerte aller Leitparameter sowie ein Vorschlag für den optimalen Zeitpunkt für die repräsentative Probennahme gehören ebenfalls zum Funktionsumfang dieser Software. Mit einer solchen Durchflußmeßzelle können Probennahmen aus Grundwassermeßstellen objektiviert, die Repräsentanz der Grundwasserproben erhöht und gleichzeitig die Abpumpzeiten gesenkt werden, was die Kosten der Probennahme reduziert.

Eine weitere vorgeschlagene Vorrichtung ist eine Bohrlochsonde zur online-Erfassung der Leitkennwerte in Grundwassermeßstellen.

Die ungespannte Grundwasserströmung wird häufig mit Hilfe der DUPUIT-Annahme idealisiert, die besagt, daß die Strömung über die gesamte Mächtigkeit des Grundwasserleiters horizontal verläuft. Reale Grundwasserströmungen weisen hingegen vertikale Strömungskomponenten auf, die durch kleine Druckunterschiede im Bereich von Zentimetern hervorgerufen werden. Damit kommt der richtigen Anlage von funktionstüchtigen Grundwassermeßstellen für eine teufenorientierte, repräsentative Probennahme eine große Bedeutung zu. Wenn eine Grundwassermeßstelle defekt ist, wird diese zwischen dem Filter und der defekten Stelle langsam durchströmt (Kurzschlußströmung). Es ist für ein 4"-Rohr bei einem Druckunterschied von nur 1 cm auf 10 m die vertikale Durchströmung des Meßstellenrohres 100.000 mal größer als die horizontale Durchströmung des Grundwasserleiters. Defekte Grundwassermeßstellen zu erkennen ist deshalb für eine repräsentative Probennahme von besonderer Bedeutung.

Als Verfahren zur direkten Messung von Kurzschlußströmungen sind Flowmetermessungen bekannt, die bei hinreichend großen Strömungsgeschwindigkeiten einsetzbar sind. Für langsame Strömungsgeschwindigkeiten werden das Bohrlochfernsehen (optische Suche nach mechanischen Beschädigungen) und Temperaturmessungen verwendet, die nicht immer zu den gewünschten Ergebnissen führen. Deshalb wird die neue Bohrlochsonde zur Lokalisierung von Wasserzutrittsstellen in einer verrohrten Bohrung auf der Basis der natürlichen Radonaktivitätskonzentration des Grundwassers vorgeschlagen.

Die Radonaktivitätskonzentration ist im Standwasser einer Grundwassermeßstelle nahezu Null, weil das Radon mit seiner Halbwertszeit von 3,8 Tagen in kurzer Zeit zerfällt. Eintrittsstellen von Grundwasser in eine Meßstelle durch defekte Muffen oder gerissene Rohre lassen sich auch bei geringsten Strömungsgeschwindigkeiten durch die Anwesenheit von Radon-222 im Standwasser der Meßstelle erkennen. Wird ein Radon-222-Log an einer Meßstelle durchgeführt, können Eintrittsstelle, Richtung der Durchströmung und je nach Strömungsgeschwindigkeit auch die Austrittsstelle von Grundwasser lokalisiert werden (Radon-222 ist sensitiv auf Grundwasser bis 15 Tage nach Eintritt in die Meßstelle). Diese Methode kann in verrohrten Bohrungen zur Lokalisierung von Kurzschlußströmungen durch defekte Muffen, undichte Rohre, funktionsuntüchtige Packer oder in mehrfach verfilterten Meßstellen eingesetzt werden und ist auch zur Lokalisierung von Wasserzutritten in unverrohrten Bohrungen geeignet. Die oben vorgeschlagene Bohrlochsonde mißt die Radonaktivitätskonzentration im Standwasser einer Meßstelle während der Befahrung online.

Hierzu wird beispielsweise ein für Radon durchlässiger Schlauch, z.B. in Form einer Spule, in das zu beprobende Wasser getaucht. Das Radon diffundiert aus dem Wasser in den Schlauch, der von Luft durchströmt wird, deren Radonkonzentration dann online in einer Meßzelle (z.B. Lucas-Zelle) gemessen werden kann.

Es besteht auch die Möglichkeit, die Radonaktivitätskonzentration als neuen Parameter in herkömmliche Durchflußmeßzellen zu integrieren. Eine derartige Durchflußmeßzelle ist in der Lage, neben der Radonaktivitätskonzentration die üblichen Sofortparameter Leitfähigkeit, Temperatur und den pH-Wert zu messen und darüberhinaus eine induktive Durchflußmessung zu realisieren. Unter Einsatz mikroelektronischer Bauelemente können alle Informationen gespeichert werden, optisch online dargestellt, protokolliert und vor allem reproduzierbar die optimale Abpumpzeit aus dem Anstieg aller Kurven ermittelt werden.

Die Erfindung ist nicht beschränkt auf die hier beschriebenen Ausführungsbeispiele. Vielmehr ist es möglich, durch geeignete Kombination der genannten Mittel und Merkmale weitere Ausführungsvarianten zu realisieren, ohne den Rahmen der Erfindung zu verlassen.

Bezugszeichenliste

1       Filterkies
1a      Filterrohr
2       Standrohr
3       Tonsperre
4       Pumpe
5       Probenahmegefäß
5a      Hals
6       Einleitungsrohr
7       Probenahmegefäßboden
9       Glasaufsatz
10      Entlüftungsrohr
11      Grundwasserprobe
12      Polyethylenschlauch
13      Schlauchkupplung
14      Cocktail

**Patentansprüche**

1.  Verfahren zur Bestimmung der optimalen Abpumpzeiten von Grundwassermeßstellen zur Festlegung des Zeitpunktes für eine repräsentative Probennahme,
    **dadurch gekennzeichnet, daß**

    -   während des Abpumpvorganges in definierter zeitlicher Aufeinanderfolge Wasserproben entnommen werden,
    -   von den entnommenen Proben die Radonaktivitätskonzentration gemessen wird und
    -   das Erreichen einer im wesentlichen konstant bleibenden Radonaktivitätskonzentration den optimalen Zeitpunkt für eine repräsentative Probennahme von Grundwasser aus dem Grundwasserbeobachtungsrohr anzeigt.

2.  Verfahren nach Anspruch 1,
    **dadurch gekennzeichnet,**
    **daß** zusäzlich zur Messung der Radonaktivitätskonzentration die elektrische Leitfähigkeit und/oder pH-Wert und/oder Temperatur gemessen wird.

3.  Verfahren nach Anspruch 1,
    **dadurch gekennzeichnet,**
    **daß** die Messung der Radonaktivitätskonzentration off-Line erfolgt derart, daß

    -   die Wasserprobe ohne Luftkontakt in ein Probenahmegefäß gefördert wird,
    -   in dem Probenahmegefäß ein Toluen-Szintillator angeordnet ist,
    -   der Toluen-Szintillator von der Wasserprobe unterschichtet wird,
    -   nachfolgend das Radon durch Schütteln des Probenahmegefäßes extrahiert und
    -   der Toluen-Szintillator-Cocktail abpipettiert und in ein Vial eingebracht wird und nachfolgend
    -   die Messung der Vials zur Bestimmung der Radonaktivitätskonzentration mit einem Flüssigszintillationsspektrometer erfolgt.

4.  Verfahren nach Anspruch 3,
    **dadurch gekennzeichnet,**

**daß** zur Erhöhung der Zählraten bei geringen Radonaktivitätskonzentrationen nach Cocktailnachfüllung mehrfach abpipettiert und in das selbe Vial abgefüllt wird.

**5.** Verfahren nach Anspruch 3,
**dadurch gekennzeichnet,**
**daß** die Beurteilung der Richtigkeit der Meßergebnisse durch Alpha/Beta-Diskriminierung erfolgt.

**6.** Verfahren nach Anspruch 1 oder 3,
**dadurch gekennzeichnet,**
**daß** die Radonkonzentration zum Zeitpunkt der Probennahme unter Berücksichtigung der Halbwertszeit des Radons durch Mehrfachmessung mit anschließender Regression bestimmt wird.

**7.** Verfahren nach Anspruch 1,
**dadurch gekennzeichnet, daß**
zusätzlich zu der Radonaktivitätskonzentration die Aktivitätskonzentrationen der Radonfolgeprodukte gemessen und die Gesamtaktivitätskonzentration bestimmt wird, wobei die Radonaktivitätskonzentration einen großen Beitrag zur Gesamtaktivitätskonzentration liefert und andere Nuklide mit größeren Halbwertszeiten das Verfahren nicht stören.

**8.** Verfahren nach einem der Ansprüche 1 oder 7,
**dadurch gekennzeichnet,**
**daß** die Messung der Radonaktivitätskonzentration und/oder der Gesamtaktivitätskonzentration on-Line erfolgt.

**9.** Verfahren nach Anspruch 8,
**dadurch gekennzeichnet,**
**daß** aus dem zu untersuchenden Grundwasser vor Ort die Radonaktivitätskonzentration und/oder die Gesamtaktivitätskonzentration spektrometrisch und/oder durch Diffusion in ein Luftvolumen oder Ausgasen und nachfolgender Messung erfaßt und ausgewertet wird.

**10.** Verfahren nach Anspruch 9,
**dadurch gekennzeichnet,**
**daß** die spektrometrische Messung durch $\alpha$-spektrometrische Bestimmung von Radon und/oder Po-218 erfolgt.

**11.** Verfahren nach Anspruch 9,
**dadurch gekennzeichnet,**
**daß** in den Förderstrom einer Pumpe ein aus einer diffusionsdurchlässigen Membran bestehender luftgefüllter Schlauch in Form einer Spule eingebracht wird, in dem das Radon aus dem Wasser hineindiffundiert und das nunmehr in der Luft enthaltene Radon in einer Meßzelle online gemessen wird.

**12.** Verfahren nach Anspruch 9,
**dadurch gekennzeichnet,**
**daß** zusätzlich zu der Erfassung der Radonaktivitätskonzentration und/oder der Gesamtaktivitätskonzentration die Parameter elektrischer Leitwert, und/oder Temperatur und/oder pH-Wert erfaßt werden.

**13.** Verfahren zur online-Erfassung der Leitkennwerte in Grundwassermeßstellen mit Hilfe einer Durchflußmeßzelle, wobei neben der Messung der natürlichen Radonaktivitätskonzentration und/oder der Gesamtaktivitätskonzentration auch die traditionellen Leitkennwerte elektrische Leitfähigkeit, Temperatur und pH-Wert im Förderstrom einer Pumpe beim Abpumpen einer Grundwassermeßstelle erfaßt werden, wobei darüberhinaus auch eine Volumenmessung integriert ist, die gemessenen Leitkennwerte Radonaktivitätskonzentration, elektrische Leitfähigkeit, Temperatur und pH-Wert kontinuierlich erfaßt und als Funktion des Abpumpvolumens durch ein zugehöriges Software-Programm online dargestellt und dokumentiert werden, wobei die Auswertung der aufgezeichneten Kurven und die Bestimmung des Plateauwertes der Aktivitätskonzentration sowie des optimalen Zeitpunktes für die repräsentative Probennahme auf der Basis eines mathematischen Algorithmus ebenfalls zum Funktionsumfang gehören.

**14.** Verfahren nach Anspruch 13,
**dadurch gekennzeichnet,**
**daß** die Messung der Radonaktivitätskonzentration und/oder der Gesamtaktivitätskonzentration über einen aus einer diffusionsdurchlässigen Membran bestehenden luftgefüllten Schlauch erfolgt.

**15.** Verfahren nach Anspruch 14,
**dadurch gekennzeichnet,**
**daß** der Schlauch in Form einer Spule ausgebildet ist.

**16.** Verwendung von Radon als natürlichem Tracer zur Erkennung und Lokalisierung von Defekten oder Kurzschlüssen an Grundwassermeßstellen, indem die Radonaktivitätskonzentration oder die Gesamtaktivitätskonzentration hinsichtlich des Erreichens eines Plateauwertes an dieser Grundwassermessstelle ausgewertet wird.

**Claims**

**1.** A method for the determination of the optimum pumping-out times of groundwater measuring sites for establishing the time for taking a representative sample, **characterized in that**

- water samples are taken during the pumping-out process in a defined temporal sequence,
- the radon activity concentration of the samples taken is measured and
- the attainment of a radon activity concentration, which remains essentially constant, indicates the optimum time for taking a representative sample of groundwater from the groundwater observation pipe.

**2.** The method of claim 1, **characterized in that**, in addition to the measurement of the radon activity concentration, the electrical conductivity and/or the pH and/or the temperature is/are also measured.

**3.** The method of claim 1, **characterized in that** the measurement of the radon activity concentration offline is carried out **in that**

- the water sample, without contacting air, is pumped into the sampling vessel,
- there is a toluene scintillator in the sampling vessel,
- the water sample is layered below the toluene scintillator,
- the radon is subsequently extracted by shaking the sampling vessel and
- the toluene scintillator cocktail is pipetted off, transferred to a vial and subsequently
- the vial is measured with a liquid scintillation spectrometer for determining the radon activity concentration.

**4.** The method of claim 3, **characterized in that**, to increase the count rates when the radon activity concentrations are low, the vial is topped up with cocktail, which is pipetted off repeatedly and filled into the same vial.

**5.** The method of claim 3, **characterized in that** the correctness of the results of the measurement is evaluated by alpha/beta discrimination.

**6.** The method of claims 1 or 3, **characterized in that** the radon concentration at the time of the sampling is determined, taking into consideration the half-life time of the radon, by carrying out repeated measurements followed by a regression analysis.

**7.** The method of claim 1, **characterized in that**, in addition to the radon activity concentration, the activity concentration of the radon daughter products is measured and the total activity concentration determined, the radon activity concentration making a large contribution to the total activity concentration and the other nuclides, which have a longer half-life time, not interfering with the method.

**8.** The method of claims 1 or 7, **characterized in that** the radon activity concentration and/or the total activity concentration are/is measured online.

**9.** The method of claim 8, **characterized in that** the radon activity concentration and/or the total activity concentration of the groundwater to be investigated is determined in situ and evaluated spectrometrically and/or by diffusion into an air volume or by outgassing and subsequent measurement.

**10.** The method of claim 9, **characterized in that** the spectrometric measurement is carried out by the $\alpha$-spectrometric determination of radon and/or Po-218.

**11.** The method of claim 9, **characterized in that** a tube, which is filled with air, consists of a membrane permeable by diffusion and is in the form of a coil and into which the radon can diffuse from the water, is brought into the output stream of a pump and that the radon, now contained in the air, is measured online in a measurement cell.

**12.** The method of claim 9, **characterized in that**, in addition to determining the radon activity concentration and/or the total activity concentration, the parameters of electrical conductivity and/or of temperature and/or of pH are determined.

**13.** A method for the online determination of the guiding characteristic values in groundwater measuring sites using a flow-through measurement cell, wherein, in addition to the measurement of the natural radon activity concentration and/or the total activity concentration, also the traditional guiding characteristic values of electrical conductivity, temperature and pH being determined in the output stream of a pump during the pumping-out of a groundwater measuring site are detected, wherein furthermore a volume measurement is integrated, the measured guiding characteristic values of radon activity concentration, electrical conductivity, temperature and pH are continuously detected and displayed and documented online by an associated software program as a function of the pumping-out volume, wherein the evaluation of the recorded curves and the determination of the plateau value of the activity concentration as well as the optimum time for taking a representative sample on the basis of a mathematical algorithm also belong to the scope of the function.

**14.** The method of claim 13,
**characterized in that**
the measurement of the radon activity concentration and/or the total activity concentration is carried out by means of an air-filled tube, consisting of a membrane, which is permeable by diffusion.

**15.** The method of claim 14,
**characterized in that**
said tube is constructed in the form of a coil.

**16.** The use of radon as a natural tracer for the identification and localization of defects or short circuits at groundwater measuring sites, wherein the radon activity concentration and/or the total activity concentration is evaluated with respect to reaching a plateau value at this groundwater measuring site.

**Revendications**

**1.** Procédé de mesure du temps de pompage optimal sur les points de mesure des eaux souterraines destiné à déterminer le moment d'obtention d'un échantillon représentatif,
**caractérisé** en ceci que

- des échantillons d'eau sont prélevés l'un après l'autre pendant le pompage, à intervalles de temps défini,
- la concentration de radon actif est mesurée sur les échantillons qui ont été prélevés,
- l'obtention d'une concentration de radon actif constant pour l'essentiel représente le moment optimal pour le prélèvement d'un échantillon représentatif de l'eau souterraine sur le tube d'observation de l'eau souterraine.

**2.** Procédé selon la revendication 1,
**caractérisé** en ceci que
en plus de la mesure de la concentration de radon actif, la conductance électrique et/ou le pH et/ou la température sont mesurés.

**3.** Procédé selon la revendication 1,
**caractérisé** en ceci que
la mesure de la concentration de radon actif est exécutée hors ligne de manière que

- l'échantillon d'eau est transféré dans une éprouvette d'échantillonnage sans contact avec l'air,
- un scintillateur au toluène est installé dans l'éprouvette d'échantillonnage,
- l'échantillon d'eau vient prendre place sous le scintillateur au toluène,
- le radon est ensuite extrait en agitant l'éprouvette avec échantillon et
- le mélange à l'intérieur du scintillateur au toluène est prélevé à la pipette pour être transféré dans un flacon et ensuite

- le contenu du flacon sert pour mesurer la concentration de radon actif par spectrométrie à scintillation en phase liquide.

**4.** Procédé selon la revendication 3,
**caractérisé** en ceci que
que pour accroître les taux de comptage pour les concentrations faibles de radon actif, la solution est prélevée à la pipette plusieurs fois après avoir complété le mélange et transférée dans le même flacon.

**5.** Procédé selon la revendication 3,
**caractérisé** en ceci que
le jugement de véracité des résultats de mesure est exécuté par discrimination alpha-bêta.

**6.** Procédé selon la revendication 1 ou 3,
**caractérisé** en ceci que
la concentration de radon au moment du prélèvement de l'échantillon est établie par mesure multiple compte tenu de la valeur de demi-vie du radon avec régression consécutive.

**7.** Procédé selon la revendication 1,
**caractérisé** en ceci que
en plus de la concentration de radon actif, les concentrations de produits de dégradation du radon actif et la concentration totale de produit actif sont établies, la concentration de radon actif réalisant un apport plus grand à la concentration totale de produit actif et les autres nuclides de demi-vie plus longue ne falsifiant pas la procédure.

**8.** Procédé selon l'une des revendications 1 ou 7,
**caractérisé** en ceci que
la mesure de la concentration de radon actif et/ou de la concentration totale de produit actif est exécutée en ligne.

**9.** Procédé selon la revendication 8,
**caractérisé** en ceci que
la concentration de radon actif et/ou la concentration totale de produit actif dans les eaux souterraines devant être analysées est saisie et analysée sur place par spectrométrie et/ou diffusion dans un volume d'air ou dégazage suivie de mesure.

**10.** Procédé selon la revendication 9,
**caractérisé** en ceci que
la mesure spectrométrique est réalisée par mesure spectrométrique $\alpha$ du radon et/ou Po-218.

**11.** Procédé selon la revendication 9,
**caractérisé** en ceci que
un tuyau flexible en forme de bobine rempli d'air consistant en une membrane perméable à la diffusion est placé dans le courant de refoulement d'une pompe, le radon de l'eau diffusant dans celui-ci et le radon alors contenu dans l'air étant mesuré en ligne réel dans une cellule de mesure.

**12.** Procédé selon la revendication 9,
**caractérisé** en ceci
que la mesure porte, en plus de la saisie de la concentration de radon actif et/ou de la concentration totale de produit actif, sur les paramètres de conductance électrique et/ou de température et/ou de pH.

**13.** Procédé de saisie des valeurs caractéristiques en ligne sur points de mesure des eaux souterraines au moyen d'une cellule de mesure de débit,
la mesure de la concentration de radon actif naturel et/ou de la concentration totale de produit actif étant complétée par la mesure des caractéristiques traditionnelles de conductance électrique, de température et de pH sur le courant de refoulement d'une pompe pendant le pompage d'un point de mesure des eaux souterraines, comportant en plus la mesure volumique, les caractéristiques mesurées de concentration de radon actif, de conductance électrique, de température et de pH étant saisies en continu et représentées et enregistrées en ligne par un programme logiciel approprié sous forme de fonction du volume de pompage, l'analyse des courbes enregistrées et la détermination de la valeur de plateau de la concentration d'activité et du moment optimal pour un prélèvement d'échantillon représentatif sur la base d'un algorithme mathématique représentant elle aussi un élément de la fonction.

**14.** Procédé selon la revendication 13,
**caractérisé** en ceci que
la mesure de la concentration de radon actif et/ou de la concentration totale de produit actif est réalisée au moyen d'un tuyau flexible rempli d'air consistant en une membrane perméable à la diffusion.

**15.** Procédé selon la revendication 14,
**caractérisé** en ceci que
le tuyau flexible a la forme d'une bobine.

**16.** Utilisation du radon comme marqueur naturel pour l'identification et la localisation des défauts et des courts-circuits sur les points de mesure des eaux souterraines par analyse d'établissement de la valeur de plateau de la concentration de radon actif ou la concentration totale de produit actif sur ce point de mesure.

Fig. 1

Fig. 2

Fig. 3

Fig. 4